(19) Europäisches Patentamt
European Patent Office
Office européen des brevets

(11) Publication number: **0 139 444**
**A1**

(12) # EUROPEAN PATENT APPLICATION

(21) Application number: 84306087.2

(22) Date of filing: 06.09.84

(51) Int. Cl.⁴: C 07 C 85/06
C 07 C 87/50

(30) Priority: 09.09.83 US 530839

(43) Date of publication of application: 02.05.85 Bulletin 85/18

(84) Designated Contracting States: DE GB

(71) Applicant: XEROX CORPORATION
Xerox Square - 020
Rochester New York 14644(US)

(72) Inventor: Szabo, Paul
61 Richview Road-Apt. 711
Islington Ontario M5A 4M8(CA)

(72) Inventor: Freeman, Daniel
2020 S. Millway 63
Mississauga Ontario L5L 1K2(CA)

(72) Inventor: Wright, Joseph D.
2164 1 Sideroad
Burlington Ontario L7R 3X4(CA)

(72) Inventor: Martin, Trevor I.
502 Shannon Crescent
Burlington Ontario I7L 2R8(CA)

(72) Inventor: Jutan, Arthur
351 Woodworth Drive
Ancaster Ontario L9G 2M9(CA)

(74) Representative: Goode, Ian Roy et al,
European Patent Attorney c/o Rank Xerox Limited Patent Department Rank Xerox House 338 Euston Road
London NW1 3BH(GB)

(54) **Continuous process for the synthesis of unsymmetrical, substituted diphenylamines.**

(57) A continuous process for the synthesis of unsymmetrical, substituted diarylamines in which a mixture of a soluble catalyst dissolved in a substituted phenol and a primary arylamine are reacted at an elevated temperature and pressure in a reactor to form a reaction mixture comprising a substituted, unsymmetrical diamine, a diphenylamine, catalyst, unreacted primary arylamine, unreacted substituted phenol, water and ammonia, which is flash separated in a flash separator to a vapor phase containing the unreacted primary arylamine, unreacted substituted phenol, water and ammonia components while substituted, unsymmetrical diamine and diphenylamine components are maintained in a liquid phase. The vapor phase is removed from the flash separator, subjected to reduced pressure, cooled and fed into a second flash separator where the water and ammonia components are flash separated to vapor phase while the unreacted primary arylamine and unreacted substituted phenol are maintained in the liquid phase. The unreacted primary arylamine and unreacted substituted phenol are then removed from the second flash evaporator and recycled in the continuous process.

EP 0 139 444 A1

# CONTINUOUS PROCESS FOR THE SYNTHESIS OF UNSYMMETRICAL, SUBSTITUTED DIPHENYLAMINES

This invention relates to a continuous process for the synthesis of unsymmetrical, substituted diphenylamines.

Procedures for the production of symmetrical, unsubstituted diphenylamines are known. Aniline is normally condensed in the vapor phase in the presence of a catalyst. Such processes are disclosed, for example in US Patents 3,079,439 and 3,118,944. The production of diphenylamine from aniline in the liquid phase is disclosed, for example, in US Patent 3,205,265 wherein the reaction is catalyzed by a halogen and an oxygen containing compound of phosphorous.

It has also been disclosed in US Patent 1,885,355 that a symmetrical, unsubstituted diamine such as di-beta-naphthol p-phenylene diamine can be prepared by reacting a diamine such as p-phenylene diamine with an excess of an unsubstituted fused benzene ring reactant such as beta-napthol at 300°C in the absence of a catalyst.

More recently, there has been disclosed a process for the manufacture of diarylamines by the reaction of a phenol and aniline in the presence of a small concentration of sulfonic acid. Such process is disclosed in German Patent 2,042,774. This patent suggests the manufacture of both symmetrical and unsymmetrical substituted diphenylamines in the presence of the acid catalyst. A continuous process is suggested through the use of interconnected autoclaves in succession whereby each autoclave is outfitted with a distillation column for the removal of water produced in the reaction. According to this process, the reaction operates with an excess of aniline. Under these conditions, the self-condensation of aniline leads to the formation of a substantial amount of by-product which is diphenylamine.

In yet another process disclosed in Canadian Patent No. 1,005,462, there is described a process for the production of diarylamines by the reaction of naphthol and a primary arylamine. In such process, ferric chloride is utilized as a catalyst. As in similar prior art, a primary arylamine is utilized in excess and formation of a by-product from the self-condensation of the primary arylamine is significant.

Generally, there is in the prior art relating to unsymmetrical, substituted diphenylamines a great tolerance for impurities in the final product, usually diphenylamine resulting from the self-condensation of aniline. The presence of such an impurity was probably of little concern because the end use of the product did not require high purity. However, there is a need to provide unsymmetrical, substituted diarylamines in high yield and purity, particularly when such amines are utilized as precursors for other products.

In accordance with the present invention, there is provided a continuous process for the synthesis of unsymmetrical, substituted diarylamines by forming a mixture of a soluble catalyst dissolved in a substituted phenol having the general formula:

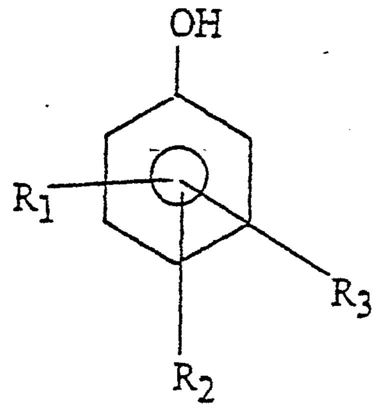

wherein $R_1$, is selected from the group consisting of methyl, ethyl, n-propyl, iso-propyl, n-butyl, sec-butyl, tert-butyl, phenyl and substituted phenyl, said substituted phenyl having attached to the benzene ring an alkyl or phenyl, and $R_2$ and $R_3$ are selected from the group consisting of hydrogen, methyl, ethyl, n-propyl, iso-propyl, n-butyl, sec-butyl, tert-butyl, phenyl and substituted phenyl, said substituted phenyl having attached to the benzene ring an alkyl, phenyl, or hydroxy group and
a primary arylamine having the general formula:

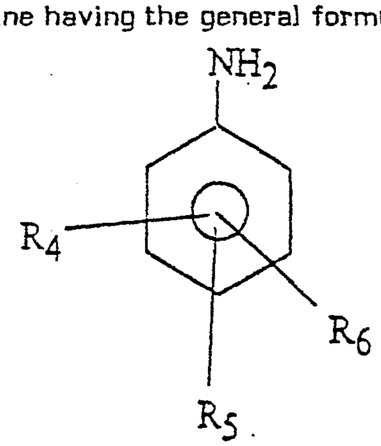

wherein $R_4$, $R_5$ and $R_6$ are selected from the group consisting of hydrogen, methyl, ethyl, n-propyl, iso-propyl, n-butyl, sec-butyl, tert-butyl, phenyl and alkyl substituted phenyl.

The mixture is heated at an elevated pressure in a reactor to form a reaction mixture comprising a substituted, unsymmetrical diamine, a diphenylamine, catalyst, unreacted primary arylamine, unreacted substituted phenol, water and ammonia. This reaction mixture is passed to a flash evaporator where the unreacted primary arylamine, unreacted substituted phenol, water and ammonia are separated in the vapor phase while the substituted, unsymmetrical diamine and diphenylamine are maintained in the liquid phase. The liquid phase containing the substituted unsymmetrical diamine product is continuously removed from the flash evaporator. The vapor phase is also continuously removed from the flash evaporator, cooled in a heat exchanger and the water and ammonia components are flash separated in a second flash evaporator while the unreacted primary arylamine and unreacted substituted phenol are maintained in the liquid phase. The unreacted primary arylamine and unreacted substituted phenol are continuously removed from the second flash evaporator and recycled in the continuous process.

The process of this invention successfully achieves the continuous removal of water and other reaction products from a complex reaction mixture based on partial condensation and evaporation without unduly lowering the reaction rate and also minimizes undesirable by-product formation due to the self-condensation of the arylamine. Moreover, low one pass conversion of the substituted phenol results in high overall conversion of the substituted phenol by the continuous recirculation of unreacted substituted phenol.

The substituted phenolic compounds of greater interest are mono-, di- or tri-substituted phenols having the general formula:

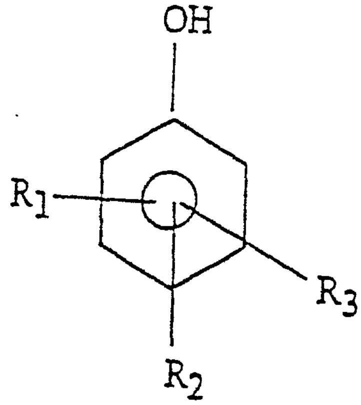

wherein $R_1$, $R_2$ and $R_3$ are defined above. $R_2$ and $R_3$ may be the same group or different groups. Typical phenolic groups include ortho-cresol, meta-cresol, para-cresol, 2,3 dimenthyl phenol, 2,4 dimethyl phenol, 2,5 dimethyl phenol, 2,6 dimethyl phenol, 3,5 dimethyl phenol, 3,4 dimethyl phenol, catechol, hydroquinone, resorcinol, p,p-biphenol, O,O-biphenol, O-hydroxy diphenyl, trimethyl phenol, dimethyl ethyl phenol, and the like. The process of this invention is particularly effective for the conversion of m-cresol to 3-methyl-diphenylamine because of the favorable yields and overall process performance.

Various substituted or unsubstituted primary arylamines can be employed in the process of this invention. These primary arylamines have the general formula:

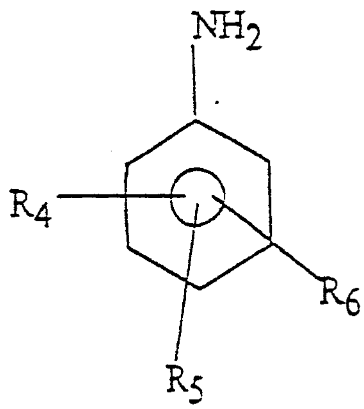

wherein $R_4$, $R_5$ and $R_6$ are defined above. $R_4$, $R_5$ and $R_6$ may be the same group or different groups. Typical substituted arylamines include aniline, ortho-toluidine, meta-toluidine, para-toluidine, 4-amino-3'-methyl biphenyl, 2,3-dimethyl aniline, 2,4-dimethyl aniline, 3,5-dimethyl aniline, 2,5-methyl ethyl aniline, 2,3,4-trimethyl aniline, 2,3-dimethyl-5-ethyl aniline and the like. For the synthesis of 3-methyldiphenylamine, aniline is preferred because of its availability and low cost.

The reaction of the primary arylamine and the substituted phenol to form the unsymmetrical, substituted diarylamine is illustrated below:

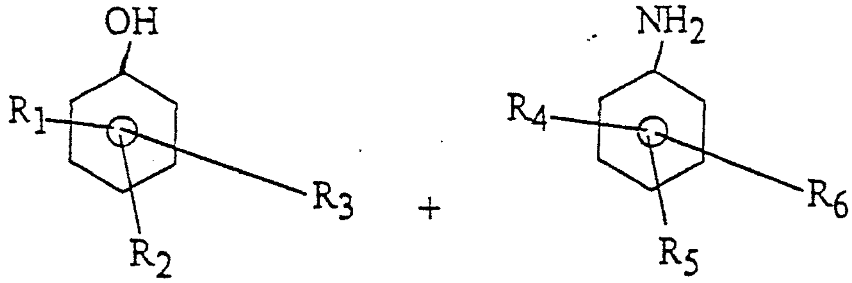

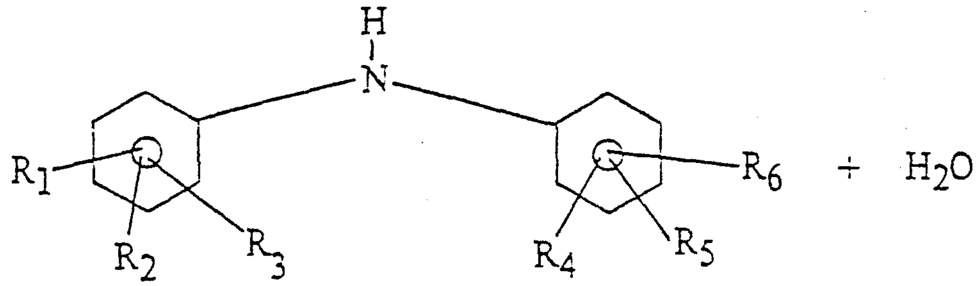

where $R_1$, $R_2$, $R_3$, $R_4$, $R_5$ and $R_6$ are defined above.

Generally, the molar ratio of primary arylamine to the phenol compound in the range between about 1:1 to about 1:10 is satisfactory because it prevents the undesired self-condensation of the arylamine. However, a molar ratio of primary arylamine to phenolic compound between about 1:1 to about 1:5 is preferred for reasons of greater reactor productivity and higher energy efficiency. At molar ratios below about 1:1, excess amine will push the reaction towards undesirable amine self-condensation. A molar ratio of primary arylamine to phenolic compound between about 1:2 to about 1:4 has been found to be optimum based on the kinetics of the reaction. The excess phenolic compound is subsequently readily recovered from the reaction mixture for reuse in the continuous production of the desired unsymmetrical, substituted diarylamine. By utilizing a substituted phenol recycling step, a high yield and overall conversion is achieved even though lower conversion is experienced in each individual pass through the reactor.

A catalyst has been found to be necessary in the process of this invention to increase the rate of reaction between the phenolic compound and the primary amine. Any suitable catalyst may be employed. Typical catalysts include, for example, ferric chloride, benzene-sulfonic acid, toluene sulfonic acid, sulfuric acid, acid alumina and the like. The amount of catalyst utilized is usually in the range of from about 1 to about 15 percent by weight based on the total weight of both reactants, although about 5 percent by weight has been found to be most efficient. A ferric chloride catalyst is preferred because of its solubility characteristics and its catalytic performance. When a partially soluble catalyst such as ferric chloride is employed, the catalyst concentration is self-controlled since it can be fed into the system as a saturated solution in the substituted phenol, the saturated concentration being a constant at a constant temperature.

Since the reaction temperature is higher than the boiling temperatures of the reactants, the reaction should be conducted under sufficient elevated pressure up to about 49 $Kg.cm^{-2}$ to keep all components in the liquid phase. Pressures between about 10.5 $Kg.cm^{-2}$ and about 49 $Kg.cm^{-2}$ in the continuous process are normally required by the thermodynamic characteristics of the system.

Reaction temperatures of between about 200°C and about 400°C provide satisfactory yields and reaction rates. The reaction rate at temperatures below about 200°C is undesirably low. Temperatures above about 400°C severely tax the capabilities of reactors. Temperatures between about 280°C and about 350°C are preferred for greater reaction rates. Optimum results are achieved with reaction temperatures between about 300°C and about 350°C because this range provides the best conversion rate.

The process of this invention will be further described with respect to the attached drawings wherein FIGS 1, 2 and 3 each represent partial views of a diagrammatic representation of the overall process of this invention.

In FIG. 1, a primary arylamine such as aniline is pumped from a feed tank (not shown) by a metering pump 10 at a desired flow rate such as about 1.8 kilograms per hour. Flow rate is monitored by a flow transmitter 11.

A substituted phenol such as m-cresol containing sufficient dissolved catalyst such as about 4 percent by weight ferric chloride is pumped from a feed tank (not shown) by a metering pump 12 shown in FIG. 1 at a desired flow rate such as about 6.1 kilograms per hour. Flow rate is monitored by a flow transmitter 13.

The two metering pumps 10 and 12 are controlled by any suitable and conventional means such as computer controlled pneumatic actuators so as to maintain the desired mole ratio between the two reactants such as a mole ratio of aniline to m-cresol of about 1:3. The aniline stream from metering pump 10 illustrated in FIG. 1 joins the m-cresol stream from metering pump 12, and the resulting stream is fed to and mixed in a static, i.e. motionless, mixer 14 shown in FIG. 2. The connecting link between the metering pumps 10 and 12 and static mixer 14 is designated by the letter

"A" in FIGS. 1 and 2. The resulting homogenized mixture then flows through two preheaters 16 and 18. The temperature at entry into the bottom of continuous reactor 20 shown in FIG. 2 is maintained at a desired reaction temperature such as about 330°C by controlling the flow of hot oil (represented by arrows) supplied by a closed loop system (not shown) through the shell side of preheaters 16 and 18 illustrated in FIG. 2.

The reactor 20 may be a cylindrical tube of sufficient size to allow the required residence time to reach the desired degree of conversion, for example, 63.5 millimeters in diameter and 2,500 millimeters in length to provide a total volume of about 7.87 litres, a total residence time of about 1 hour, and a degree conversion of the amine of about 90%. An inert atmosphere such as nitrogen is introdudced at 21 (represented by a hollow arrow) at the bottom of reactor 20 shown in FIG. 2. The reaction is highly endothermic. The reactor 20 is heated by means of an electrical heating jacket with three zones independently controlled via control loops 22, 24, and 26 so as to maintain the desired reaction temperature at about 330°C along the length of the reactor 20. The temperature in the reactor is monitored by means of temperature transmitters 28, 29, 30, 31 and 32. The rate of flow through the clyindrical tube reactor 20 is selected so as to achieve a laminar flow which allows for a true "plug flow regime". The expression "plug flow regime" is defined as the flow pattern in which all the material in the reactor flows at the same velocity, irrespective of its radial distance from the center of the reactor. Alternatively, the reactor 20 may be utilized as a mixed reactor with different catalytic systems such as heterogeneous (suspended catalyst) or packed bed catalyst systems. However these latter catalytic systems are less preferred because of the necessity of periodically recharging the catalyst. If desired, other types of reactors such as continuous multistage tubular, continuous stirred tank, continuous stirred tank cascade or continuous recirculation reactor systems may be substituted for the continuous cylindrical tube reactor 20 illustrated in FIG. 2. A continuous cylindrical tube reactor is preferred because of its simplicity, high continuous throughput characteristics, and residence time distribution characteristics.

The pressure in reactor 20 is maintained at a level corresponding

to the vapor pressure of the reaction mixture at the reaction temperature such about 35 $Kg.cm^{-2}$. The reactor pressure is monitored by means of a pressure transducer 34 located at the top of reactor 20. The reactor pressure is maintained by the control valve 36 shown in FIG. 3.

The liquid mixture leaving the reactor 20 contains the substituted, unsymmetrical diphenylamine reaction product such as 3-methyldiphenylamine, by-products such as diphenylamine, unreacted substituted phenol such as m-cresol, unreacted primary arylamine such as aniline, and volatile components formed in the reaction such as water and ammonia.

The mixture stream passes through a control valve 36 illustrated in FIG. 3 controlled by the process control computer to flash the mixture to a lower pressure such as about 4.9 $Kg.cm^{-2}$. The connecting link between reactor 20 and control valve 36 is designated by the letter "B" in FIGS. 2 and 3. The temperatures of the mixture stream prior to and after passage through control valve 36 are monitored by means of temperature transmitters 38 and 40, respectively. The pressure of the mixture stream after passage through control valve 36 is also maintained in flash separator 42 by the control valve 52 such as to provide the right temperature/pressure pair to allow the desired split of components between the vapor and the liquid phase. The pressure in flash separator 42 is monitored by means of a pressure transducer 44. The temperature in flash separator 42 is maintained at a level calculated by computer based on liquid vapor equilibrium information so as to maintain all components in the vapor phase except the reaction product and by-product such as 3-methyldiphenylamine and diphenylamine, respectively, which are the heaviest components with the highest boiling points in the mixture. A temperature, for example, of about 300°C can be maintained in flash separator 42 by a conventional temperature control loop using hot oil. Temperature of the materials in the flash separator 42 is monitored by means of a temperature transmitter 45. Any required additional heat (latent heat) is supplied to flash separator 42 by heating coil 46 through which hot oil is circulated. Circulation of the hot oil is regulated by a suitable conventional control loop, such as constant temperature/variable flow.

The liquid level in flash separator 42 is controlled by a

conventional level switch (not shown) which controls the liquid exit valve 47 such as to maintain the desired level. The liquid level in flash separator 42 is monitored by means of a level transmitter 48. The liquid containing the reaction product and by-product is passed through a cooler 50 and collected in a suitable storage tank (not shown) at a rate, for example, of about 3.2 kilograms per hour. The cooler 50 is cooled by conventional means such as a water jacket.

The vapor phase from flash separator 42 passes through a control valve 52 shown in FIG. 3 where the pressure is further reduced to a level set by the computer via vapor liquid equilibrium calculation. The temperature of the vapor phase after it passes through control valve 52 is monitored by means of a temperature transmitter 54. The vapor phase is thereafter fed into water cooled condenser 56 and into flash separator 58 shown in FIG. 3. The pressure and temperature in flash separator 58 is controlled so as to maintain in the liquid phase only the unreacted unsubstituted phenol such as m-cresol and unreacted primary arylamine such aniline. For example, a suitable pressure and temperature for the specific conditions described above will be about $3.15 Kg.cm^{-2}$ at about 210°C, respectively. The temperature of the material withdrawn from cooler 56 is monitored by means of temperature transmitter 60. The pressure in flash separator 58 is monitored by means of a pressure transducer 62. Temperature of the materials in the flash separator 58 is monitored by means of a temperature transmitter 64. Any required additional cooling is supplied to flash separator 58 by cooling coil 66 through which a suitable cooling medium such as water is circulated. The liquid level in flash separator 58 is monitored by means of a level transmitter 68. The liquid level in flash separator 58 is controlled by a conventional level switch (not shown) which controls the liquid exit valve 70 at the side of flash separator 58 such as to maintain the desired level. The liquid removed from the flash separator 58 containing unreacted primary arylamine and unreacted substituted phenol is passed through a water cooled cooler 72 and collected in a suitable tank not shown from which the unreacted substituted phenol can be recycled via metering pumps not shown at a suitable rate such as about ~4.0 kilograms per hour. The volatile components such as water and ammonia leaving flash separator 58

as vapor are condensed in water cooled condenser 74. Volatile components such as ammonia may not totally condense and, in such case, a suitable absorption system not shown may be utilized for disposing of the ammonia.

In European Patent publication 0027004 published on April 15, 1981, a reaction is described in which aniline and m-cresol are reacted in the presence of a ferric chloride catalyst. The process is a batch process in which a control valve is utilized to periodically vent the system allowing water to be removed during the reaction as it accumulates. As the reaction proceeds, the pressure oscillates in the narrow range of the control valve setting as the water is removed but is maintained between about $10.5 Kg.cm^{-2}$ and $17.5 Kg.cm^{-2}$. If desired, the water may be removed continuously rather than periodically. At the end of the reaction, the reactants are cooled and the entire contents of the reactor are transferred to a vacuum distillation system. The catalyst may be removed by filtration prior to distillation. The first fraction contains water and traces of m-cresol and aniline. This is discarded and a second fraction is obtained containing 98% m-cresol with traces of aniline, diphenylamine and 3-methyldiphenylamine. This fraction is reused as the m-cresol for the next batch. The residue from the vacuum distillation is then treated with sodium tripolyphosphate and held to 100°C for about 1 hour with stirring and thereafter filtered to remove inorganic salts and the filtrate is transferred through a second vacuum distillation system. A small amount of potassium carbonate may be added to the second distillation system to produce a colorless product. An intermediate fraction is obtained from the second vacuum distillation system containing traces of m-cresol, aniline, diphenylamine and a slight amount of 3-methyldiphenylamine. This intermediate fraction may be combined with subsequent batches for recovery of useful material therein. The main fraction is recovered at a temperature in the range of from about 170°C to about 180°C under $10^{-2}$mm of mercury vacuum to provide 3-methyldiphenylamine in excess of 97% purity. A small amount of diphenylamine is also present and the diphenylamine content of the final product is dependent upon the efficiency of the distillation unit. The residue is removed from the second distillation system with water. The excess phenolic compound is recycled to provide a higher yield and overall conversion while at the same time

providing a highly pure product.

As described above, a process for preparing a symmetrical secondary aromatic amine at 300°C in the absence of a catalyst is disclosed by Jones in US Patent 1,885,355. In an example in line 73, page 1 through line 2, page 2, of the Jones patent, 400 pounds of 2-naphthol and 100 pounds of p-phenylene diamine are reacted at 300°C for a period of 2 hours to form a crude containing 2-naphthol and di-beta-naphthol p-phenylene diamine. The crude is comminuted into a fine powder and washed with 115 gallons of hot denatured alcohol followed by 25 gallons of cold alcohol to provide a pure product of 98% yield. Self-condensation of the p-phenylene diamine does not take place in the Jones reaction and the mole ratio does not affect self-condensation of the diamine.

When about 5 moles of aniline and about 16.6 moles of m-cresol and no catalyst are used in the foregoing process described in European Patent publication 0027004, no significant amount of 3-methyldiphenylamine could be detected by liquid and gas chromatography at the end of the reaction. This clearly demonstrates that the reaction will not proceed in the absence of a catalyst. The aforesaid European Patent publication 0027004 also indicates that the degree of self-condensation of aniline is dramatically affected by the ratio of primary aromatic amine and substituted phenol employed. Moreover, since the reactants will boil away at temperatures above their boiling points, and since the reaction must be effected at temperatues above the boiling points of the reactants, the reaction cannot be successfully conducted at atmospheric pressure. Thus the process described in European Patent publication 0027004 is clearly distinct from the process of Jones in reactants, catalyst, conditions and results.

Similarly, the invention of the instant application can be distinguished from the process described in European Patent publication 0027004 in that the invention of the instant application is continuous and eliminates the very expensive and energy intensive step of separation by distillation of a complex reaction mixture. Moreover, unlike process described in European Patent publication 0027004, the invention of the instant application utilizes the available energy of the reaction mixture stream to operate the system under partial conversion/high selectivity

without the significant energy drawbacks of such a type of operation. In addition, the most important parameters such as conversion rate, selectivity (ratio between the reaction product and the self-condensation product of two moles of amine) are easily controlled and varied within a large domain by simply changing the feed rates via the metering pumps. Moreover, the removal from the system of the volatile components formed during the reaction, e.g. water and ammonia, enhances the reaction rate while occuring as an intrinsic part of the process flow. Further, a process model based on the system pressure can be easily formulated to control all the important reaction parameters such as degree of conversion and selectivity without the need for complicated on-line composition measurements.

The invention will now be described in detail with respect to specific preferred embodiments thereof, it being understood that these examples are intended to be illustrative only and the invention is not intended to be limited to the materials, conditions, process parameters, and the like recited herein. All parts and percentages are by weight unless otherwise indicated.

EXAMPLE I

A reaction system arrangement similar to that illustrated in FIGS. 1-3 was used to prepare 3-methyldiphenylamine. Two metering pumps were employed to maintain a mole ratio between aniline and m-cresol of about 1:3. Sufficient ferric chloride was added to the stream of m-cresol to form a saturated solution. These reactant streams from the metering pumps were joined and mixed in a static mixer. The resulting homogenized mixture was then preheated to raise the temperature of the mixture to about 330°C. The heated mixture was introduced into the bottom of a reator maintained at a reaction temperature of about 330°C. The reactor was a cylindrical tube of about 63.5 millimeters in diameter and 2,500 millimeters in length to provide a total volume of about 7.87 litres. The flow rate of the reactants was adjusted to achieve a total residence time of about 1 hour, and a degree conversion of the aniline of about 95 percent. The degree of conversion is determined via material balance over several hours of continuous operation. An inert atmosphere of nitrogen was maintained in the reactor prior to start-up. Since the reaction was

endothermic, the reactor was heated by means of an electrical heating jacket with three zones independently controlled via control loops so as to maintain the desired reaction temperature at about 330°C along the length of the reactor. The temperature in the reactor was monitored by means of temperature transmitters arranged along the length of the reactor. The rate of flow through the cylindrical tube reactor was adjusted so as to achieve a laminar flow which allowed for a true "plug flow regime". Achievement of laminar flow was assured keeping the linear velocity at very low levels. The pressure in the reactor was maintained at a level corresponding to the vapor pressure of the reaction mixture at the reaction temperature which was about $35 Kg.cm^{-2}$. The reactor pressure was monitored by means of a pressure transducer located at the top of reactor. The reactor pressure was maintained by the pressure control loop controlling the expansion valve. The liquid mixture leaving the top of the reactor contained the substituted, unsymmetrical diphenylamine reaction product 3-methyldiphenylamine, by-product diphenylamine, unreacted m-cresol, unreacted aniline, and volatile components formed in the reaction such as water and ammonia. The liquid mixture was passed through a control valve to flash the mixture to a lower pressure of about $7 Kg.cm^{-2}$. The temperature of the mixture stream prior to the control valve was about 330°C and was about 300°C after passage through control valve. After flashing to the lower pressure, the mixture stream was introduced into a flash separator. The pressure of the mixture stream after passage through control valve was also maintained in the flash separator by the second pressure control loop. The temperature in the flash separator was maintained at about 300°C by the temperature control loop feeding hot oil into the heating coil such as to maintain all components in the vapor phase except the reaction product and by-product 3-methyldiphenylamine and diphenylamine, respectively, which were the heaviest components with the highest boiling points in the mixture. Additional heat was supplied to the flash separator by a heating coil through which hot oil was circulated. Circulation of the hot oil was regulated by the above mentioned control loop. The liquid level in the flash separator was controlled by a level switch which controlled a liquid exit valve. The liquid containing the reaction product and by-product was passed through a cooler and collected

in a suitable storage tank at a rate of about 2.0 kilograms per hour. The vapor phase from the flash separator was passed through a control valve where the pressure was reduced to about 3.15Kg.cm$^{-2}$. The temperature of the vapor phase after it passed through the control valve was about 250°C. The vapor phase was thereafter transported to a water cooled condenser and into a second flash separator. The pressure and temperature in the second flash separator was about 3.15Kg.cm$^{-2}$ at 210°C, respectively to maintain in the liquid phase only the unreacted unsubstituted phenol m-cresol and unreacted primary arylamine aniline. Additional cooling was supplied to the second flash separator by a cooling coil through which water was circulated. The liquid removed from the second flash separator containing unreacted primary arylamine and unreacted substituted phenol was passed through a water cooled cooler and collected in a tank from which the unreacted substituted phenol was recycled via metering pumps not shown at a rate of about 4.0 kilograms per hour. The volatile components such as water and ammonia leaving the second flash separator were condensed in a water cooled condenser.

EXAMPLE II

A reaction system arrangement similar to that illustrated in FIGS. 1-3 is believed to be capable of preparing 3,4-dimetyldiphenylamine. Two metering pumps can be employed to maintain a mole ratio between aniline and 3,4-dimethylphenol of about 1:3. Sufficient ferric chloride should be added to the stream of 3,4-dimethylphenol to form a saturated solution. These reactant streams from the metering pumps can be joined and mixed in a static mixer. The resulting homogenized mixture can then be preheated to raise the temperature of the mixture to about 310°C. The heated mixture should be introduced into the bottom of a reactor maintained at a reaction temperature of about 310°C. The reactor can be a cylindrical tube of about 63.5 millimeters in diameter and 2,500 millimeters in length to provide a total volume of about 7.87 litres. The flow rate of the reactants should be adjusted to achieve a total residence time of about 1.5 hours and a degree conversion of the aniline of about 95 percent. The degree of conversion may be determined via material balance over several hours of continuous operation. An inert atmosphere of nitrogen can be introduced at the bottom of reactor. Since the reaction is

highly endothermic, the reactor can be heated by means of an electrical heating jacket with three zones independently controlled via control loops so as to maintain the desired reaction temperature at about 310°C along the length of the reactor. The temperature in the reactor can be monitored by means of temperature transmitters arranged along the length of the reactor. The rate of flow through the cylindrical tube reactor is adjusted so as to achieve a laminar flow which allows for a true plug flow regime. Achievement of laminar flow can be assured by keeping the linear velocity at very low levels. The pressure in the reactor was maintained at a level corresponding to the vapor pressure in the reactor mixture at the reaction temperature. The reactor pressure can be monitored by means of a pressure transducer located at the top of reactor. The liquid mixture leaving the top of the reactor will contain the substituted, unsymmetrical diphenylamine reaction product 3,4-dimethyldiphenylamine, by-product diphenylamine, unreacted 3,4-dimethylphenol, unreacted aniline and volatile components formed in the reaction such as water and ammonia. The liquid mixture can then be passed through a control valve to flash the mixture to a lower pressure. After flashing to the lower pressure, the mixture stream can then be introduced into a flash separator. The pressure of the mixture stream after passage through control valve can also be maintained in the flash separator by the pressure contol loop. The temperature in the flash separator can be maintained by control loops such as to maintain all components in the vapor phase except the reaction product and by-product 3,4-dimethyldiphenylamine and dimethylamine, respectively, which are the heaviest components with the highest boiling points in the mixture. Additional heat can be supplied to the flash separator by a heating coil through which hot oil is circulated. Circulation of the hot oil can be regulated by a control loop. The liquid level in the flash separator can be controlled by a level switch which controls a liquid exit valve. The liquid containing the reaction product and by-product is passed through a cooler and collected in a suitable storage tank. The vapor phase from the flash separator is passed through a control valve where the pressure is further reduced. The vapor phase is thereafter transported to a water cooled condenser and into a second flash separator. The pressure and temperature in the second flash separator is maintained such as to

maintain in the liquid phase only the unreacted substituted phenol, 3,4-dimethylphenol, and unreacted primary arylamine, aniline. Additional cooling is supplied to the second flash separator by a cooling coil through which water is circulated. The liquid removed from the second flash separator containing unreacted primary arylamine and unreacted substituted phenol is passed through a water cooled cooler and collected in a tank from which the unreacted substituted phenol is recycled. The volatile components such as water and ammonia leaving the second flash separator is condensed in a water cooled condenser.

EXAMPLE III

A reaction system arrangement similar to that illustrated in FIGS. 1-3 is believed capable of preparing 2-isopropyl, 3-methyldiphenylamine. Two metering pumps can be employed to maintain a mole ratio between 2-isopropyl aniline and m-cresol of about 1:3. Sufficient ferric chloride should be added to the stream of m-cresol to form a saturated solution. These reactant streams from the metering pumps are joined and mixed in a static mixer. The resulting homogenized mixture is then preheated to raise the temperature of the mixture to about 300°C. The heated mixture is introduced into the bottom of a reactor maintained at a reaction temperature of about 300°C. The reactor is a cylindrical tube of about 63.5 millimeters in diameter and 2,500 millimeters in length to provide a total volume of about 7.87 litres. The flow rate of the reactants is adjusted to achieve a total residence time of about 1.5 hours. A degree conversion of the amine of approximately 90 percent is believed to be attainable. Since the reaction is endothermic, the reactor is heated by means of an electical heating jacket with three zones independently controlled via control loops so as to maintain the desired reaction temperature at about 300°C along the length of the reactor. The temperature in the reactor is monitored by means of temperature transmitters arranged along the length of the reactor. The rate of flow through the cylindrical tube reactor is adjusted so as to achieve a laminar flow which allows for a true plug flow regime. The pressure in the reactor is maintained at a level coresponding to the vapor pressure of the reaction mixture at the reaction temperature. The reactor pressure is monitored by means of a pressure transducer located at the top of reactor. The reactor

pressure is maintained by the pressure control loop. The liquid mixture leaving the top of the reactor contains the substituted, unsymmetrical diphenylamine reaction produt 2-isopropyl, 3-methyldiphenylamine, by-product 2,2'-diisopropydiphenylamine, unreacted m-cresol, unreacted 2-isopropyl aniline, and volatile components formed in the reaction such as water and ammonia. The liquid mixture is passed through a control valve to flash the mixture to a lower pressure. After flashing to the lower pressure, the mixture stream is introduced into a flash separator. The pressure of the mixture stream after passage through control valve is also maintained by the pressure control loop. The temperature in the flash separator is maintained by control loops such as to maintain all components in the vapor phase except the reaction product and by-product, which are the heaviest components with the highest boiling points in the mixture. Additional heat is supplied to the flash separator by a heating coil through which hot oil is circulated. Circulation of the hot oil is regulated by a temperature control loop. The liquid level in the flash separator is controlled by a level switch which controls a liquid exit valve. The liquid containing the reaction product and by-product was passed through a cooler and collected in a suitable storage tank. The vapor phase from the flash separator is passed through a control valve where the pressure is further reduced. The vapor phase is thereafter transported to a water cooled condenser and into a second flash separator. The pressure and temperature in the second flash separator is controlled such as to maintain in the liquid phase only the unreacted substituted phenol and unreacted primary arylamine. Additional cooling is supplied to the second flash separator by a cooling coil through which water is circulated. The liquid removed from the second flash separator containing unreacted primary arylamine and unreacted substituted phenol is passed through a water cooled cooler and collected in a tank from which the unreacted substituted phenol is recycled. The volatile components such as water and ammonia leaving the second flash separator can be condensed in a water cooled condenser.

EXAMPLE IV

A reaction system arrangement similar to that illustrated in FIGS. 1-3 is believed to be capable of preparing 2-t-butyl, 6-methyl-4'-n-butyldiphenylamine. Two metering pumps can be employed to maintain a

mole ratio between 2-t-butyl, 6-methylaniline and 4-n-butylphenol of about 1:3. Sufficient ferric chloride should be added to the stream of 4-n-butylphenol to form a saturated solution. These reactant streams from the metering pumps are joined and mixed in a static mixer. The resulting homogenized mixture is then preheated to raise the temperature of the mixture to about 300°C. The heated mixture is introduced into the bottom of a reactor maintained at a reaction temperature of about 300°C. The reactor can be a cylindrical tube of about 63.5 millimeters in diameter and 2,500 millimeters in length to provide a total volume of about 7.87 litres. The flow rate of the reactants can be adjusted to achieve a toal residence time of about 1.5 hours. A degree conversion of the amine of approximately 90 percent is believed to be attainable. An inert atmosphere of nitrogen was introduced at the bottom of reactor. Since the reaction is endothermic, the reactor is heated by means of an electrical heating jacket with three zones independently controlled via control loops so as to maintain the desired reaction temperature at about 300°C along the length of the reactor. The temperature in the reactor is monitored by means of temperature transmitters arranged along the length of the reactor. The rate of flow through the cylindrical tube reactor is adjusted so as to achieve a laminar flow which allows for a true plug flow regime. The pressure in the reactor is maintained at a level corresponding to the vapor pressure of the reaction mixture at the reaction temperature. The reactor presure is monitored by the pressure control loop. The liquid mixture leaving the top of the reactor should contain the substituted, unsymmetrical diphenylamine reaction product, self-condensation by-product, unreacted amine, unreacted phenol, and volatile components formed in the reaction such as water and ammonia. The liquid mixture is passed through a control valve to flash the mixture to a lower pressure. After flashing to the lower pressure, the mixture stream is introduced into a flash separator. The pressure of the mixture stream after passage through control valve is also maintained in the flash separator by the second pressure control loop. The temperature in the flash separator is maintained by control loops to maintain all components in the vapor phase except the reaction product and by-product, which are the heaviest components with the highest boiling points in the mixture. Additional heat

is supplied to the flash separator by a heating coil through which hot oil is circulated. Circulation of the hot oil is regulated by a temperature control loop. The liquid level in the flash separator is controlled by a level switch which controls a liquid exit valve. The liquid containing the reaction product and by-product is passed through a cooler and collected in a suitable storage tank. The vapor phase from the flash separator is passed through a control valve where the pressure is further reduced. The vapor phase is thereafter transported to a water cooled condenser and into a second flash separator. The pressure and temperature in the second flash separator is controlled such as to maintain in the liquid phase only the unreacted unsubstituted phenol and the unreacted primary arylamine. Additional cooling is supplied to the second flash separator by a cooling coil through which water is circulated. The liquid removed from the second flash separator containing unreacted primary arylamine and unreacted substituted phenol is passed through a water cooled cooler and collected in a tank from which the unreacted substituted phenol is recycled. The volatile components such as water and ammonia leaving the second flash separator are condensed in a water cooled condenser.

Claims:

1. A continuous process for the synthesis of unsymmetrical, substituted diphenylamines comprising forming a mixture of a catalyst dissolved in a substituted phenol having the general formula:

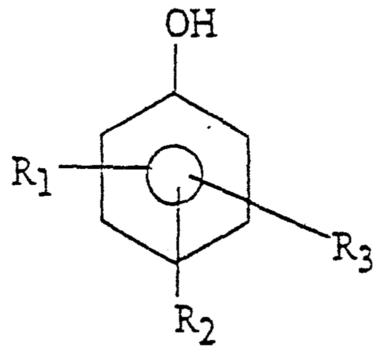

wherein $R_1$ is selected from the group consisting of methyl, ethyl, n-propyl, iso-propyl, n-butyl, sec-butyl, tert-butyl, phenyl and substituted phenyl, said substituted phenyl having attached to the benzene ring an alkyl or phenyl group and
$R_2$ and $R_3$ are selected from the group consisting of hydrogen, methyl, ethyl, n-propyl, iso-propyl, n-butyl, sec-butyl, tert-butyl, phenyl and substituted phenyl, said substituted phenyl having attached to the benzene ring an alkyl, phenyl, or hydroxy group and
a primary arylamine having the general formula:

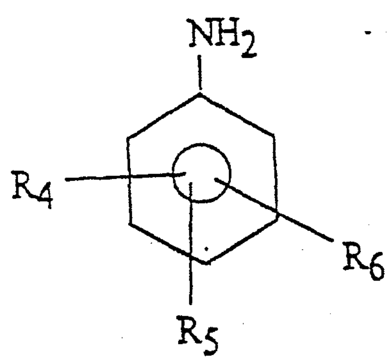

wherein $R_4$, $R_5$ and $R_6$ are selected from the group consisting of hydrogen, methyl, ethyl, n-propyl, iso-propyl, n-butyl, sec-butyl, tert-butyl, phenyl and alkyl substituted phenyl

and heating said mixture at an elevated pressure in a continuous

reactor to form a reaction mixture comprising a substituted, unsymmetrical diamine, a diphenylamine, catalyst, unreacted primary arylamine, unreacted substituted phenol, water and ammonia, removing said reaction mixture from said reactor, flash separating in a flash separator to a vapor phase said unreacted primary arylamine, unreacted substituted phenol, water and ammonia while maintaining said substituted, unsymmetrical diamine, and reaction by-product in a liquid phase, removing said liquid phase from said flash separator, removing said vapor phase from said flash separator, cooling said vapor phase in a second flash separator after further reducing the pressure, flash separating to a vapor phase said water and ammonia while maintaining said unreacted primary arylamine and said unreacted substituted phenol in a liquid phase, and removing said unreacted primary arylamine and said unreacted substituted phenol from said second flash separator.

2. A process according to Claim 1 including recycling at least a portion of said unreacted substituted phenol removed from said second flash separator for reaction in said continuous reactor.

3. A process according to Claim 1 wherein said primary arylamine is aniline and said substituted phenol is meta cresol.

4. A process according to Claim 1 wherein said catalyst is ferric chloride dissolved in said substituted phenol to form a saturated solution prior to combining said primary arylamine with said substituted phenol.

5. A process according to Claim 1 wherein the molar ratio of primary arylamine to substituted phenol is in the range of about 1:1 to about 1:10.

6. A process according to Claim 1 including heating said mixture in said continuous reactor to temperature between about 200°C and about 400°C.

7. A process according to Claim 1 including maintaining said

elevated pressure in said continuous reactor at the vapor pressure of said reaction mixture at the reaction temperature, up to about $49Kg.cm^{-2}$.

8. A process according to Claim 1 including maintaining said elevated pressure in said continuous reactor between about $10.5Kg.cm^{-2}$ and about $49Kg.cm^{-2}$.

9. A process according to Claim 1 including flashing said reaction mixture from said continuous reactor to a lower pressure prior to flash separating said reaction mixture in said flash separator.

10. A process according to Claim 1 including flashing said vapor phase from said flash separator to a lower pressure prior to cooling and feeding said vapor phase into a second flash separator.

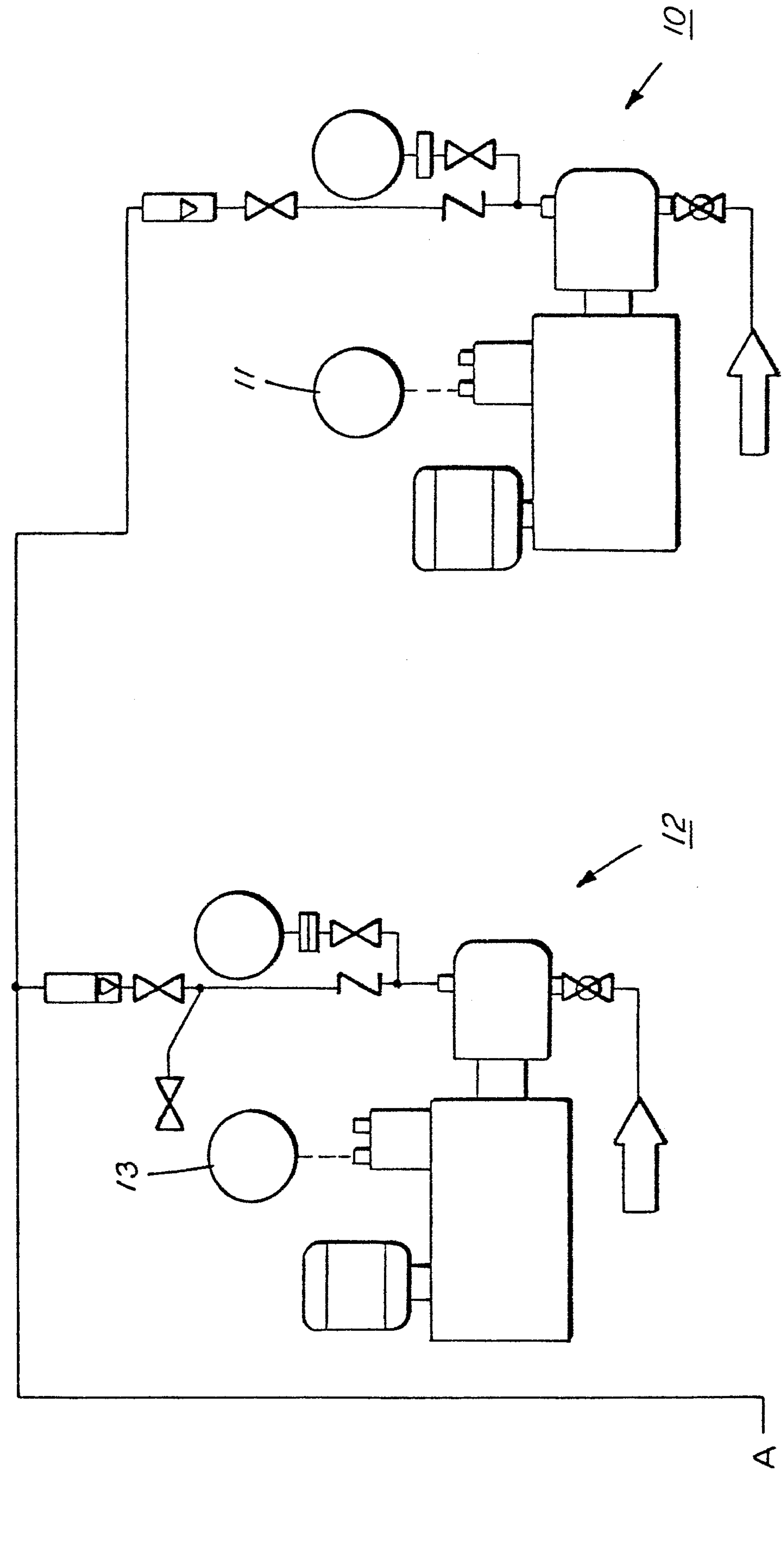


FIG. 1

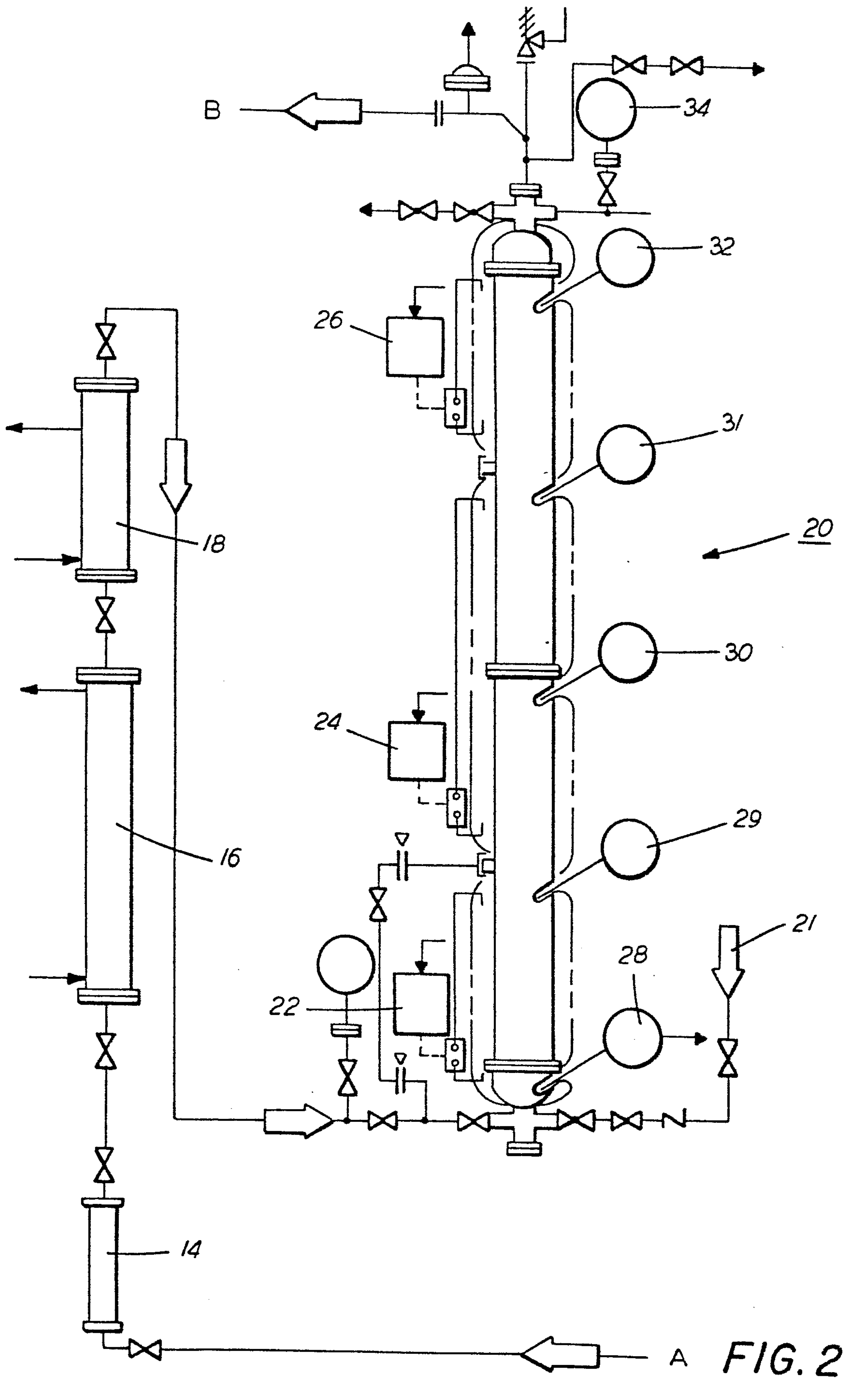


FIG. 2

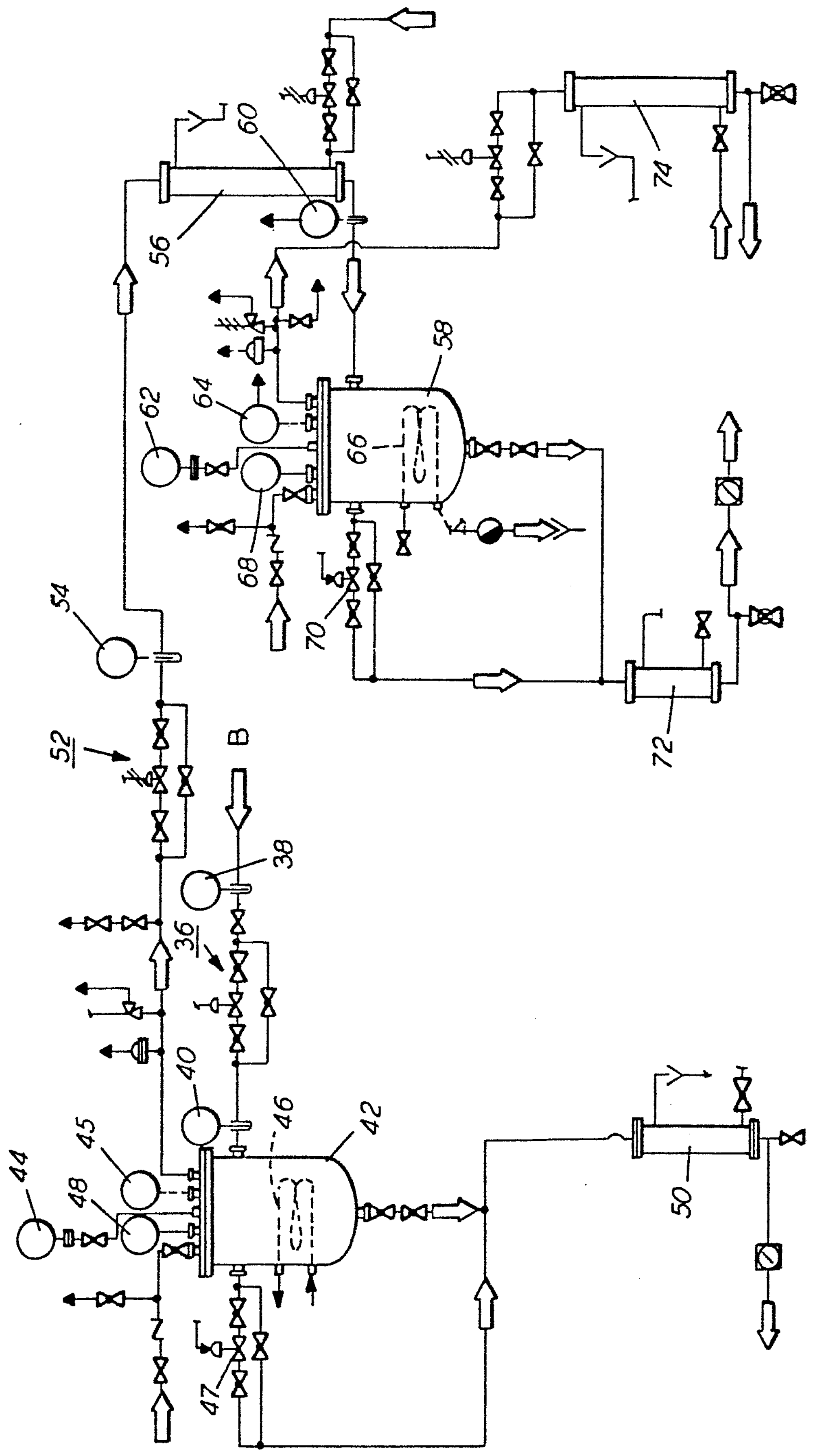


FIG. 3

European Patent Office

# EUROPEAN SEARCH REPORT

0139444

Application number

EP 84306087.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP - A1 - 0 027 004 (XEROX CORPORATION)<br>* Page 3, line 27 - page 5, line 15; claims 1,2,4-10 *<br>-- | 1-8 | C 07 C 85/06<br>C 07 C 87/50 |
| D,A | DE - A - 2 042 774 (BILLER)<br>* Example 7; page 6, line 25 - page 7, line 16 *<br>-- | 1,3,5-8 | |
| A | DE - A - 2 307 233 (AMERICAN CYANAMID)<br>* Page 4, lines 6-24; example 1 * | 1,6,7 | |
| D,A | & CA-A-1 005 462<br>-- | | |
| A | GB - A - 611 316 (TIMBROL LIMITED)<br>* Example 3 *<br>---- | 1,3,5-8 | |

TECHNICAL FIELDS SEARCHED (Int. Cl.4)

C 07 C 85/00
C 07 c 87/00

The present search report has been drawn up for all claims

| Place of search | Date of completion of the search | Examiner |
|---|---|---|
| VIENNA | 29-11-1984 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82